(19) Europäisches Patentamt
European Patent Office
Office européen des brevets

(11) **EP 4 304 474 B1**

(12) **FASCICULE DE BREVET EUROPEEN**

(45) Date de publication et mention
de la délivrance du brevet:
**02.07.2025 Bulletin 2025/27**

(21) Numéro de dépôt: **22720477.3**

(22) Date de dépôt: **07.04.2022**

(51) Classification Internationale des Brevets (IPC):
**A61B 5/11** *(2006.01)* **A61B 5/103** *(2006.01)*

(52) Classification Coopérative des Brevets (CPC):
**A61B 5/112; A61B 5/1038; A61B 5/1121;
A61B 5/7267**

(86) Numéro de dépôt international:
**PCT/FR2022/050657**

(87) Numéro de publication internationale:
**WO 2022/214769 (13.10.2022 Gazette 2022/41)**

(54) **PROCEDE ET DISPOSITIF D'INFERENCE D'UNE LIGNE DE PRESSION PLANTAIRE**

VERFAHREN UND VORRICHTUNG ZUM ABLEITEN EINER PLANTAREN DRUCKLINIE

METHOD AND DEVICE FOR INFERRING A PLANTAR PRESSURE LINE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **07.04.2021 FR 2103554**

(43) Date de publication de la demande:
**17.01.2024 Bulletin 2024/03**

(73) Titulaires:
• **Digitsole**
**54000 Nancy (FR)**
• **Zhor Tech**
**54000 Nancy (FR)**

(72) Inventeurs:
• **DUMOND, Rémy**
**54000 NANCY (FR)**
• **ALLAIS, Roman**
**54000 NANCY (FR)**

(74) Mandataire: **A.P.I. Conseil**
**Technopôle Hélioparc**
**4, rue Jules Ferry**
**64000 Pau (FR)**

(56) Documents cités:
**CN-A- 108 209 924 CN-A- 111 631 719
US-A1- 2009 163 834**

• **DELA CRUZ MICHAELA ANNE A ET AL: "Joint
Gait Kinematic and Kinetic Analysis using
Inertial Measurement Units and Plantar Pressure
Sensor System", 2019 IEEE 11TH
INTERNATIONAL CONFERENCE ON HUMANOID,
NANOTECHNOLOGY, INFORMATION
TECHNOLOGY, COMMUNICATION AND
CONTROL, ENVIRONMENT, AND MANAGEMENT
( HNICEM ), IEEE, 29 November 2019
(2019-11-29), pages 1 - 6, XP033760647, DOI:
10.1109/HNICEM48295.2019.9072701**
• **DING MING ET AL: "Control of Walking Assist
Exoskeleton With Time-delay Based on the
Prediction of Plantar Force", IEEE ACCESS,
IEEE, USA, vol. 8, 19 July 2020 (2020-07-19),
pages 138642 - 138651, XP011802649, DOI:
10.1109/ACCESS.2020.3010644**
• **MAHDAVIAN MOHAMMAD ET AL: "Motion
Generation of a Wearable Hip Exoskeleton Robot
Using Machine Learning-Based Estimation of
Ground Reaction Forces and Moments", 2019
IEEE/ASME INTERNATIONAL CONFERENCE ON
ADVANCED INTELLIGENT MECHATRONICS
(AIM), IEEE, 8 July 2019 (2019-07-08), pages 796 -
801, XP033630020, DOI: 10.1109/
AIM.2019.8868759**

## Description

## Domaine technique

L'invention concerne le domaine de la podométrie. En particulier, il concerne un procédé de détermination d'une ligne de pression plantaire d'un individu. L'invention concerne en outre un dispositif ou un système pour la détermination d'une ligne de pression plantaire d'un individu.

## Technique antérieure

Ci-après, nous décrivons l'art antérieur connu à partir duquel l'invention a été développée.

Le pied, comportant 26 os, 107 ligaments et près de 19 muscles, constitue une partie du corps humain particulièrement complexe. Il revêt également un rôle important puisqu'il est la clef de voûte permettant à un être humain de se mouvoir. La moindre dégradation de ce dernier peut rapidement être handicapante. Même si cela est particulièrement vrai dans le cadre de la pratique d'un sport impliquant un contact du pied avec le sol, une mauvaise démarche lors d'activités quotidienne peut ainsi avoir un impact non négligeable sur la santé. L'étude des forces appliquées sur le pied au cours de la marche est donc en constante évolution et de nouveaux systèmes ou de nouveaux indicateurs permettant de faciliter ces études voient régulièrement le jour.

Ainsi, il a été déjà proposé de suivre la démarche d'un individu au travers de capteurs de pression ou de centrale inertielles situées en particulier au niveau du pied. De récentes publications montrent notamment l'intérêt d'utiliser des centrales inertielles positionnées au niveau du pied pour accéder en temps réel à des données de démarche (WO2019077266) ou encore des données de troubles de la démarche (WO2019193301) ou de paramètres avancés de démarches (WO2020217037). Il a aussi été montré l'existence d'une corrélation entre la cinématique et la cinétique de la marche d'une personne à l'aide de capteurs portables tels que des centrales inertielles sur les membres et des capteurs de pression plantaire (Michaela Anne A. Dela Cruz et al. Joint Gait Kinematic and Kinetic Analysis using Inertial Measurement Units and Plantar Pressure Sensor System. Conference 2019 IEEE. 10.1109/HNICEM48295.2019.9072701). Il a en outre été proposé une méthode de prédiction de la force plantaire et de l'état de la marche pour éviter le retard du contrôle d'un exosquelette d'aide à la marche (MAHDAVIAN MOHAMMAD ET AL: "Motion Generation of a Wearable Hip Exoskeleton Robot Using Machine Learning-Based Estimation of Ground Reaction Forces and Moments", 2019 IEEE/ASME INTERNATIONAL CONFERENCE ON ADVANCED INTELLIGENT MECHATRONICS (AIM), 10.1109/AIM.2019.8868759). Néanmoins, aucun de ces documents ne s'intéressent à la détermination d'une ligne de pression plantaire.

Parmi la pluralité d'indicateurs concernant la démarche, la ligne de pression plantaire (« gaitline » en terminologie anglosaxonne) est un indicateur essentiel pour l'analyse des appuis d'un individu. En particulier, une ligne de pression plantaire anormale pourrait trouver son origine dans des altérations morphologiques telles que des fractures ou des microlésions osseuses, une différence de longueur d'un métatarsien ou encore un hallux-valgus mais aussi d'altérations neurologiques telles que des hémiplégies ou des maladies neurodégénératives telles que Parkinson.

La ligne de pression plantaire est étudiée depuis des années à partir de capteurs de force ou de pression. En effet, la ligne de pression plantaire est une ligne qui illustre comment une pression moyenne, un centre de pression, d'un pied change pendant le temps que le pied est en contact avec le sol. De nombreux autres documents proposent des procédés et appareils, impliquant le calcul d'une ligne de pression plantaire, pour diagnostiquer des problèmes de posture ou de mouvement corporel, en particulier de défauts ou de problèmes de position et de démarche (US20090163834) tels que ceux liés à une rupture du ligament croisé antérieur basé (CN108209924) ou ceux associés à un risque de chute d'une personne âgée (CN111631719).

Cependant, cette analyse est bien souvent effectuée par des professionnels de santé puisque celle-ci nécessite un système de mesure bien particulier et une expertise du professionnel de santé pour analyser les résultats générés par le système de mesure.

Classiquement, l'analyse de la ligne de pression plantaire, telle que pratiquée par les professionnels de santé est basée principalement sur l'étude des pressions plantaires en statique et en dynamique plus communément au travers d'études podométriques ou baropodométriques. En déclenchant des récepteurs (capteurs de pression), placés dans un tapis relié à un ordinateur, sous l'effet de l'appui du pied d'un patient, une cartographie instantanée de ces appuis est obtenue. Le signal produit par l'activation des récepteurs est recueilli et traité, puis reproduit sous forme d'image où chaque valeur de pression est convertie en couleur. Le professionnel de santé peut ainsi visualiser à quels niveaux les pressions plantaires se situent, de façon à en déduire une ligne de pression plantaire possiblement révélatrice d'une démarche altérée. De telles analyses sont essentielles au professionnel de santé puisque c'est à partir de celles-ci qu'il pourra par exemple proposer au patient des orthèses plantaires adaptées permettant de corriger la démarche du patient.

D'autres solutions ont été proposées essentiellement via l'intégration de capteurs de pression dans des semelles. En effet, l'utilisation d'accéléromètres a été considérée comme préjudiciable à la précision d'un modèle et nécessitant des capteurs supplémentaires d'un autre type pour dériver des paramètres qui nécessitent des informations sur la ligne de pression plantaire (WO2012026818). A titre d'exemple, l'étude réalisée par Janin M. « Marche athlétique : modification des pressions plantaires par éléments d'orthèse » ; Cinésiologie ; 2002:203:13-4 détaille un système d'analyse de la marche à partir des variations de pressions lors du déroulement du pas. Le matériel requis pour l'analyse des pressions au sol est un dispositif comprenant un support comportant des capteurs piézoélectriques unidirectionnels verticaux, de forme identique à une semelle et pouvant s'insérer dans la chaussure de sport afin d'enregistrer les variations de pressions lors du déroulement du pas. Ce système d'analyse est relié à un ordinateur pour l'acquisition, et est composé d'un boîtier d'enregistrement et de câbles conducteurs, d'une carte d'enregistrement télécommandée permettant l'enregistrement dynamique des appuis plantaires et de leurs répartitions. Ainsi, malgré l'absence d'un tapis spécifique pour l'analyse de la pression, le matériel ne peut pas mettre un suivi en continu de la ligne de pression ou le calcul d'une ligne de pression à partir d'activité de plein air.

Récemment, il a été proposé des chaussures comportant des capteurs de pression et capables de générer des données pouvant être traités ultérieurement pour fournir une ligne de pression plantaire d'un individu. En particulier, le signal de sortie moyenné spatialement des capteurs peut permettre la génération d'une ligne de pression plantaire dans un espace bidimensionnel. Plus particulièrement la demande de brevet N° WO2013022344 porte sur un système d'analyse de la dynamique du pied et notamment de son comportement viscoélastique pour évaluer l'efficacité d'une course, le risque de blessure ou le confort d'un utilisateur. Un tel système prend la forme d'une semelle comportant une surface sensible à la pression équipée d'une pluralité de capteurs de pression pour générer un signal électronique en fonction d'une pression exercée. En outre, un dispositif électronique est agencé dans la semelle et permet d'effectuer des mesures, en fonction du temps, d'un centre de pression mobile exercé par le pied de l'utilisateur sur la surface sensible à la pression, formant ainsi une ligne de pression plantaire le long d'un axe avant d'une direction de marche.

Néanmoins, les solutions existantes ne sont pas optimales. Elles nécessitent le déploiement de matériels couteux ou prennent la forme d'un système intégrant de nombreux dispositifs dont la mise en œuvre doit être effectuée dans des conditions spécifiques pour l'obtention de mesures pertinentes. En outre, les systèmes sensibles à la pression intégrés dans les semelles sont encore trop fragiles et couteux. Dans ces conditions, ils ne permettent pas de déterminer aisément et avec précision une marche anormale.

Il existe donc un besoin pour une nouvelle solution intégrée à une chaussure permettant une analyse dynamique de la ligne de pression plantaire de façon à permettre la mise en évidence facilité et robuste d'une marche anormale.

L'invention a pour but de remédier aux inconvénients de l'art antérieur. En particulier, l'invention a pour but de proposer un procédé permettant de déterminer rapidement, et de préférence en temps réel, une ligne de pression plantaire et cela sans la nécessité d'utiliser des équipement couteux ou fragiles. L'invention a en outre pour but de proposer un dispositif et un système permettant la détermination d'une ligne de pression dans de nombreuses situations.

## Résumé de l'invention

L'invention vise à pallier ces inconvénients.

L'invention vise en particulier un procédé d'inférence d'une ligne de pression plantaire, ladite la ligne de pression plantaire correspondant à l'évolution temporelle de la position du barycentre des pressions plantaires lors du déplacement d'un individu et comportant une pluralité de positions successives dudit barycentre des pressions plantaires pour un pied dudit individu, ledit procédé étant mis en œuvre par un ou plusieurs microprocesseurs à partir de données de mouvement générées par au moins une centrale inertielle couplée au pied dudit individu, par exemple couplée à une chaussure portée par ledit individu ou au pied dudit individu, ladite au moins une centrale inertielle étant configurée pour générer des données de mouvement pour une pluralité d'instants d'une phase d'appui d'un pied dudit l'individu, ledit procédé comportant les étapes suivantes :

- Chargement d'un modèle de corrélation, ledit modèle de corrélation définissant une relation entre une pluralité de données obtenues à partir de capteurs de pression plantaire et une pluralité de valeurs d'angle de pied par rapport à un référentiel prédéterminé, ladite relation permettant de déterminer la position du barycentre des pressions plantaires à chaque instant d'une phase d'appui ;

- Calcul de valeurs d'angles du pied par rapport au référentiel prédéterminé, à partir des données de mouvement générées par l'au moins une centrale inertielle, sur au moins deux instants de la phase d'appui du pied dudit l'individu ; et

- Inférence d'une pluralité de positions successives du barycentre des pressions plantaires pour former la ligne de pression plantaire du pied dudit individu, à partir des valeurs d'angles calculées sur les au moins deux instants de la phase d'appui du pied et du modèle de corrélation.

La demanderesse a développé un procédé capable de déterminer une ligne de pression plantaire à partir de données de mouvement générées par une centrale inertielle. Alors que la ligne de pression plantaire n'était accessible jusqu'à aujourd'hui qu'à partir de données provenant de capteurs de pression, une telle innovation permet d'élargir considérablement les cas d'usage et de suivi de la ligne de pression plantaire.

La demanderesse a développé en particulier un procédé de détermination d'une ligne de pression plantaire inférée pour un individu portant des chaussures auxquelles sont couplées des centrales inertielles. Un tel système est beaucoup plus abordable que des tapis de podométrie classiques et moins fragiles que des semelles intégrant des capteurs de pression embarqués.

Selon d'autres caractéristiques optionnelles du procédé, ce dernier peut inclure facultativement une ou plusieurs des caractéristiques suivantes, seules ou en combinaison :

- l'étape d'inférence est répétée de façon à établir la ligne de pression plantaire inférée à partir de plusieurs phases d'appui. Ainsi, la ligne de pression déterminée est une ligne de pression dynamique apportant plus d'information qu'une ligne de pression statique.

- la ou les centrales inertielles sont des centrales inertielles six axes ou neuf axes. L'étape de calcul est donc réalisée à partir de données de mouvement générées sur six axes ou neuf axes. En particulier, par des centrales inertielles six axes ou neuf axes. L'utilisation de centrales inertielles comportant gyroscope et accéléromètre est particulièrement avantageuse dans le cadre de l'invention.

- la ou les centrales inertielles sont positionnées dans une semelle amovible ou non amovible. L'étape de calcul est donc réalisée à partir de données de mouvement générées par des centrales inertielles alors qu'elles sont positionnées dans une semelle amovible ou non amovible. La position de la ou des centrales inertielles dans une semelle amovible permet notamment de s'adapter à différentes chaussures portées par l'utilisateur.

- la ou les centrales inertielles sont positionnées dans un boitier électronique agencé pour être fixé sur la chaussure portée par ledit individu. L'étape de calcul est donc réalisée à partir de données de mouvement générées par des centrales inertielles alors qu'elles sont positionnées dans un boitier électronique agencé pour être fixé sur la chaussure portée par ledit individu. En effet, la centrale inertielle peut être positionnée sur le dessus de la chaussure, par exemple au niveau de la languette, de la tige ou encore du contrefort.

- la ou les centrales inertielles sont intégrées à un dispositif électronique comportant les un ou plusieurs microprocesseurs configurés pour mettre en œuvre le procédé. L'étape de calcul est donc réalisée à partir de données de mouvement générées par des centrales inertielles alors qu'elles sont positionnées dans un boitier électronique, ledit boitier électronique comportant également les un ou plusieurs microprocesseurs configurés pour calculer les valeurs d'angle du pied par rapport au référentiel prédéterminé. De préférence, l'inférence est également mise en œuvre par les un ou plusieurs microprocesseurs disposés dans le boitiers électronique. Ainsi, le calcul est réalisé directement au niveau d'un dispositif intégrant les centrales sans nécessiter l'accès à des serveurs informatiques dédiés.

- le dispositif électronique est intégré à une semelle. Ainsi, les calcul de valeurs d'angles du pied et inférence d'une pluralité de centres de pression sont mis en œuvre par les un ou plusieurs microprocesseurs positionnés dans un boitier électronique intégré à une semelle.

- le calcul de valeurs d'angles du pied est réalisé pour au moins cinq instants de la pose du pied. Cela permet d'augmenter la justesse de la ligne de pression plantaire déterminée.

- le calcul de valeurs d'angles du pied comporte le calcul de valeurs d'au moins deux angles du pied. Cela permet d'augmenter la justesse de la ligne de pression plantaire déterminée.

- Il comporte un calcul d'une ligne de pression plantaire sans prendre en compte de données provenant d'un capteur de pression couplé à la chaussure de l'individu. Ainsi, il n'est plus nécessaire d'intégrer des capteur de pression fragiles et couteux.

- il comporte un calcul d'une ligne de pression plantaire pour chacun des pieds de l'individu. Le calcul de la ligne de pression plantaire est donc fait pour chacun des pieds de l'individu.

- il comporte une comparaison des lignes de pressions plantaires de chacun des pieds de l'individu. Cela permet d'identifier des asymétries.

- il comporte une comparaison des lignes de pressions plantaires obtenues pour un même pied pour deux intervalles temporels différents. Cela permet d'identifier une évolution dans le temps des lignes de pressions.

- le modèle de corrélation correspond à un modèle d'apprentissage automatique.

- le modèle d'apprentissage automatique est sélectionné parmi un modèle d'apprentissage automatique supervisé, non supervisé ou par renforcement. Cela permet d'augmenter la justesse de la ligne de pression plantaire déterminée.

- il comporte une étape de prétraitement des données de mouvement générées par la au moins une centrale inertielle, l'étape de prétraitement comportant un filtrage fréquentiel, une suppression de la gravité, et/ou une suppression de la dérive.

- les valeurs d'angles du pied calculées, par rapport au référentiel prédéterminé, comportent des valeurs d'angles sélectionnées parmi : un angle de l'axe antéro-postérieur du pied par rapport à sa ligne de progression, un angle de l'axe antéro-postérieur du pied par rapport au sol, un angle de l'axe transversal du pied par rapport à sa ligne de progression ou un angle de l'axe transversal du pied par rapport au sol. Cela permet d'augmenter la justesse de la ligne de pression plantaire déterminée.

- il comporte en outre une étape de transmission de la ligne de pression plantaire inférée à un terminal externe.

Selon un deuxième objet, l'invention porte sur un dispositif d'inférence d'une ligne de pression plantaire, ladite ligne de pression plantaire correspondant à l'évolution temporelle de la position du barycentre des pressions plantaires lors du déplacement d'un individu et comportant une pluralité de positions successives dudit barycentre des pressions plantaires pour un pied dudit individu, ledit dispositif d'inférence comportant un ou plusieurs microprocesseurs, lesdits un ou plusieurs microprocesseurs étant configuré pour :

- Charger un modèle de corrélation, ledit modèle de corrélation définissant une relation entre une pluralité de données obtenues à partir de capteurs de pression plantaire et une pluralité de valeurs d'angle de pied par rapport à un référentiel prédéterminé, ladite relation permettant de déterminer la position du barycentre des pressions plantaires à chaque instant d'une phase d'appui ;

- Calculer des valeurs d'angles du pied par rapport au référentiel prédéterminé, à partir de données de mouvement générées par au moins une centrale inertielle, sur au moins deux instants de la phase d'appui du pied dudit l'individu ; et

- Inférer une pluralité de positions successives du barycentre des pressions plantaires pour former la ligne de pression plantaire du pied dudit individu, à partir des valeurs d'angles calculées sur les au moins deux instants de la phase d'appui du pied et du modèle de corrélation.

Selon d'autres caractéristiques optionnelles du dispositif, ce dernier peut comporter en outre, au moins une centrale inertielle apte à être couplée à une chaussure portée par ledit individu ou au pied dudit individu, ladite au moins une centrale inertielle étant configurée pour générer des données de mouvement pour une pluralité d'instants d'une phase d'appui d'un pied dudit l'individu.

**Brève description des dessins**

D'autres caractéristiques et avantages de l'invention seront mieux compris à la lecture de la description qui va suivre et en référence aux dessins annexés, donnés à titre illustratif et nullement limitatif.

- La figure 1 représente un schéma d'un procédé selon un mode de réalisation de l'invention.

- La figure 2 représente un schéma d'un procédé selon un autre mode de réalisation de l'invention. Les étapes en pointillés sont facultatives.

- La figure 3 représente une illustration de quatre pas d'un individu, mettant en évidence une ligne de marche et des angles de pied.

- La figure 4 illustre différents positionnements de la centrale inertielle couplée à une chaussure ou à un pied.

- La figure 5 représente l'illustration de deux lignes de pression plantaire inférées par un procédé selon l'invention.

- La figure 6 représente un système comportant un dispositif d'inférence d'une ligne de pression plantaire selon l'invention.

Les figures ne respectent pas nécessairement les échelles, notamment en épaisseur, et ce à des fins d'illustration.

Des aspects de la présente invention sont décrits en référence à des organigrammes et / ou à des schémas fonctionnels de procédés, d'appareils (systèmes) et de produits de programme d'ordinateur selon des modes de réalisation de l'invention.

Sur les figures, les organigrammes et les schémas fonctionnels illustrent l'architecture, la fonctionnalité et le fonctionnement d'implémentations possibles de systèmes, de procédés et de produits de programme d'ordinateur selon divers modes de réalisation de la présente invention. A cet égard, chaque bloc dans les organigrammes ou blocs-diagrammes peut représenter un système, un dispositif, un module ou un code, qui comprend une ou plusieurs instructions exécutables pour mettre en œuvre la ou les fonctions logiques spécifiées. Dans certaines implémentations, les fonctions associées aux blocs peuvent apparaître dans un ordre différent que celui indiqué sur les figures. Par exemple, deux blocs montrés successivement peuvent, en fait, être exécutés sensiblement simultanément, ou les blocs peuvent parfois être exécutés dans l'ordre inverse, en fonction de la fonctionnalité impliquée. Chaque bloc des schémas de principe et / ou de l'organigramme, et des combinaisons de blocs dans les schémas de principe et / ou l'organigramme, peuvent être mis en œuvre par des systèmes matériels spéciaux qui exécutent les fonctions ou actes spécifiés ou effectuer des combinaisons de matériel spécial et d'instructions informatiques.

**Description des modes de réalisation**

Ci-après, nous décrivons un résumé de l'invention et le vocabulaire associé, avant de présenter les inconvénients de l'art antérieur, puis enfin de montrer plus en détail comment l'invention y remédie.

Dans la suite de la description, l'expression « ligne de pression plantaire » au sens de l'invention correspond à l'évolution de la position du centre des pressions plantaires (e.g. barycentre) lors du déplacement d'un individu (e.g. une marche ou une course), de préférence depuis la pose du talon jusqu'au décollage des orteils.

L'expression « ligne de pression plantaire inférée » ou « inférence d'une ligne de pression plantaire » au sens de l'invention correspond à une prédiction de l'évolution de la position du centre des pressions plantaires (e.g. barycentre) lors du déplacement d'un individu telle que générée à partir de données de mouvement du pied de l'individu. De façon préférée, la ligne de pression plantaire inférée correspond à une ligne de pression plantaire dynamique. C'est-à-dire qu'elle est générée au travers d'une analyse dans le temps de la répartition de la pression plantaire et en particulier du centre des pressions plantaires.

L'expression « données de mouvement » peut correspondre au sens de l'invention à des données comportant des valeurs d'accélération, de vitesse angulaire, de champs magnétiques ou encore des données de fusion obtenus à partir d'une ou plusieurs de ces valeurs. Les données de mouvements peuvent être des données brutes ou des données prétraitées.

L'expression « phase d'appui » au sens de l'invention peut correspondre, dans le cadre d'une analyse du cycle de marche ou de course, au moment où le pied est au moins en partie en contact avec le sol. Elle peut notamment comprendre l'attaque du pas, le pas antérieur, le pas postérieur, et la propulsion qui se termine par le décollement du pied.

L'expression « instant de la phase d'appui » au sens de l'invention correspond à un intervalle temporel survenant pendant la pose du pied aussi appelée phase d'appui du pied.

L'expression « cycle de marche » au sens de l'invention correspond à l'intervalle de temps se situant entre deux appuis du talon d'une même jambe sur le sol, ou plus généralement deux événements identiques répétés.

Par « modèle de corrélation », il faut comprendre au sens de l'invention une suite finie d'opérations ou d'instructions permettant de calculer une valeur à partir d'une ou plusieurs valeurs d'entrée. La mise en œuvre de cette suite finie d'opérations permet par exemple d'attribuer une valeur Y, telle qu'une étiquette Y, à une observation décrite par un ensemble de caractéristiques ou paramètres X grâce par exemple à la mise en œuvre d'une fonction f, susceptible de reproduire Y en ayant observé X.

$$Y = f\,(X) + e$$

où e symbolise le bruit ou erreur de mesure.

Par « modèle d'apprentissage automatique supervisé », on entend au sens de l'invention un modèle de corrélation généré automatiquement à partir de données, appelées observations qui ont été étiquetées.

Par « modèle d'apprentissage automatique non supervisée », on entend au sens de l'invention un modèle de corrélation généré automatiquement à partir de données, appelées observations qui n'ont pas été étiquetées.

L'expression « valeurs d'angles du pied » au sens de l'invention peut correspondre à des valeurs d'angle permettant de représenter la position d'un pied de l'individu dans son environnement, c'est-à-dire par exemple par rapport à un référentiel prédéterminé. Cette position pouvant être relative à des membres de l'individu avec par exemple l'angle formé par l'axe du tibia et l'axe antéropostérieur du pied. Cette position peut aussi être relative à des éléments extérieurs à l'individu avec par exemple l'angle formé par l'axe antéropostérieur du pied et le sol. Enfin, cette position peut aussi être relative à un angle formé par l'axe antéropostérieur du pied et une ligne de marche calculée ou une trajectoire calculée du pied.

L'expression « référentiel prédéterminé » au sens de l'invention peut correspondre à un référentiel inertiel tel qu'un repère terrestre ou un référentiel non inertiel comme un ou plusieurs membres de l'individu ou encore un repère généré à partir de données de mouvement de l'individu.

On entend par « semelle » un objet permettant de séparer le pied de l'individu du sol. Une chaussure peut comporter une couche de semelle supérieure en contact direct avec le pied de l'individu et une couche de semelle inférieure en contact direct avec le sol ou plus généralement l'environnement extérieur. Une chaussure peut aussi comporter une semelle interne amovible.

On entend par « amovible » la capacité à être détachée, enlevée ou démontée aisément sans avoir à détruire des moyens de fixation soit parce qu'il n'y a pas de moyens de fixation soit parce que les moyens de fixation sont aisément et rapidement démontables (e.g. encoche, vis, languette, ergot, clips). Par exemple, par amovible, il faut comprendre que l'objet n'est pas fixé par soudure ou par un autre moyen non prévu pour permettre de détacher l'objet.

On entend par « traiter », « calculer », « déterminer », « afficher », « transformer », « extraire », « comparer » ou plus largement « opération exécutable », au sens de l'invention, une action effectuée par un dispositif ou un processeur sauf si le contexte indique autrement. À cet égard, les opérations se rapportent à des actions et/ou des processus d'un système de traitement de données, par exemple un système informatique ou un dispositif informatique électronique, qui manipule et transforme les données représentées en tant que quantités physiques (électroniques) dans les mémoires du système informatique ou d'autres dispositifs de stockage, de transmission ou d'affichage de l'information. Ces opérations peuvent se baser sur des applications ou des logiciels.

Les termes ou expressions « application », « logiciel », « code de programme », et « code exécutable » signifient toute expression, code ou notation, d'un ensemble d'instructions destinées à provoquer un traitement de données pour effectuer une fonction particulière directement ou indirectement (e.g. après une opération de conversion vers un autre code). Les exemples de code de programme peuvent inclure, sans s'y limiter, un sous-programme, une fonction, une application exécutable, un code source, un code objet, une bibliothèque et/ou toute autre séquence d'instructions conçues pour l'exécution sur un système informatique.

On entend par « processeur », au sens de l'invention, au moins un circuit matériel configuré pour exécuter des opérations selon des instructions contenues dans un code. Le circuit matériel peut être un circuit intégré. Des exemples d'un processeur comprennent, sans s'y limiter, une unité de traitement central, un processeur graphique, un circuit intégré spécifique à l'application (ASIC) et un circuit logique programmable.

On entend par « dispositif électronique », tout dispositif comprenant une unité de traitement ou un processeur, par exemple sous la forme d'un microcontrôleur coopérant avec une mémoire de données, éventuellement une mémoire programme, lesdites mémoires pouvant être dissociées. L'unité de traitement coopère avec lesdites mémoires au moyen d'un bus de communication interne.

On entend par « couplé », au sens de l'invention, connecté, directement ou indirectement avec un ou plusieurs éléments intermédiaires. Deux éléments peuvent être couplés mécaniquement, électriquement ou liés par un canal de communication.

L'étude de la démarche des individus est en pleine expansion et les procédés de suivi et de caractérisation se multiplient. Néanmoins, certaines analyses utilisées depuis des dizaines d'années telles que l'étude du centre des pressions plantaire, nécessitent encore des équipements fragiles et couteux, limitant la démocratisation de ces études.

Alors que les capteurs de pression plantaires étaient, jusqu'à la présente invention, considérés comme un passage obligé pour la détermination d'une ligne de pression plantaire, la demanderesse a développé une solution permettant de déterminer cette ligne de pression plantaire à partir de données provenant d'une centrale inertielle.

Ainsi, selon un premier aspect, l'invention concerne un procédé 1 d'inférence d'une ligne de pression plantaire 30.

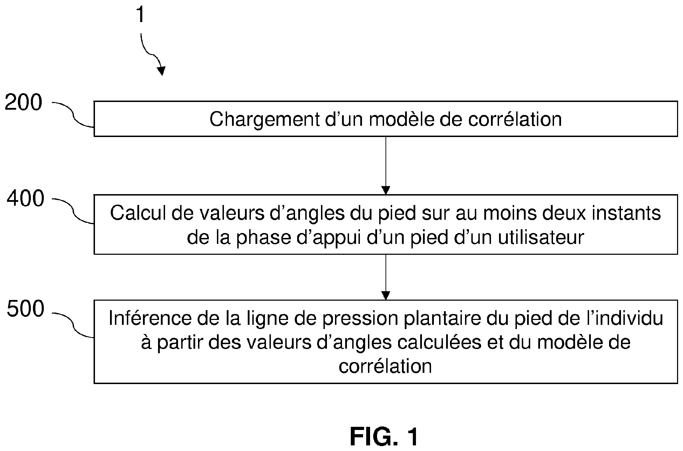

La figure 1 illustre un mode de réalisation de ce procédé d'inférence 1.

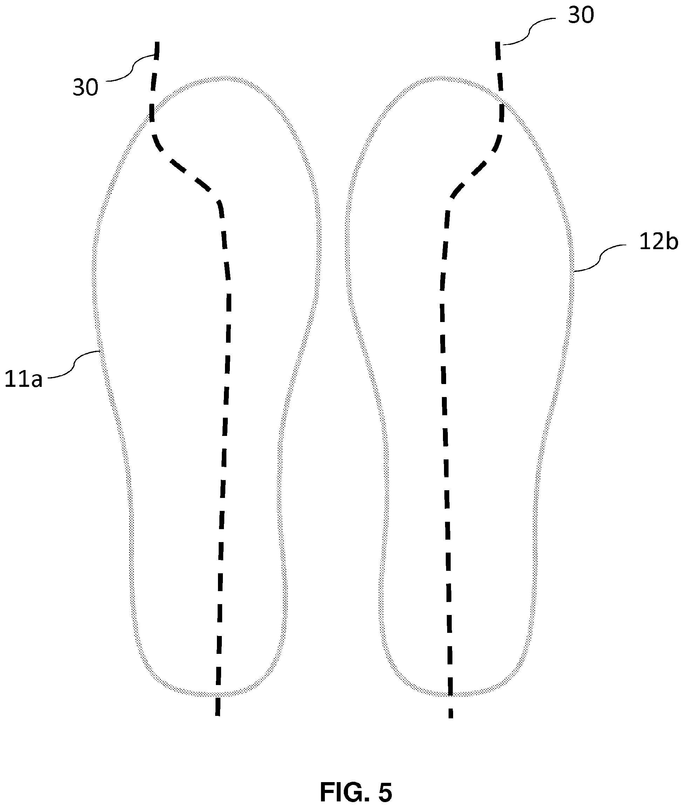

Comme illustré, un procédé de détermination d'une ligne de pression plantaire 30 (illustrée à la figure 5) inférée selon l'invention comportera un chargement 200 d'un modèle de corrélation, un calcul 400 de valeurs d'angles d'un pied sur au moins deux instants de la phase d'appui du pied d'un individu ; et une inférence 500 de la ligne de pression plantaire du pied de l'individu à partir des valeurs d'angles calculées et du modèle de corrélation.

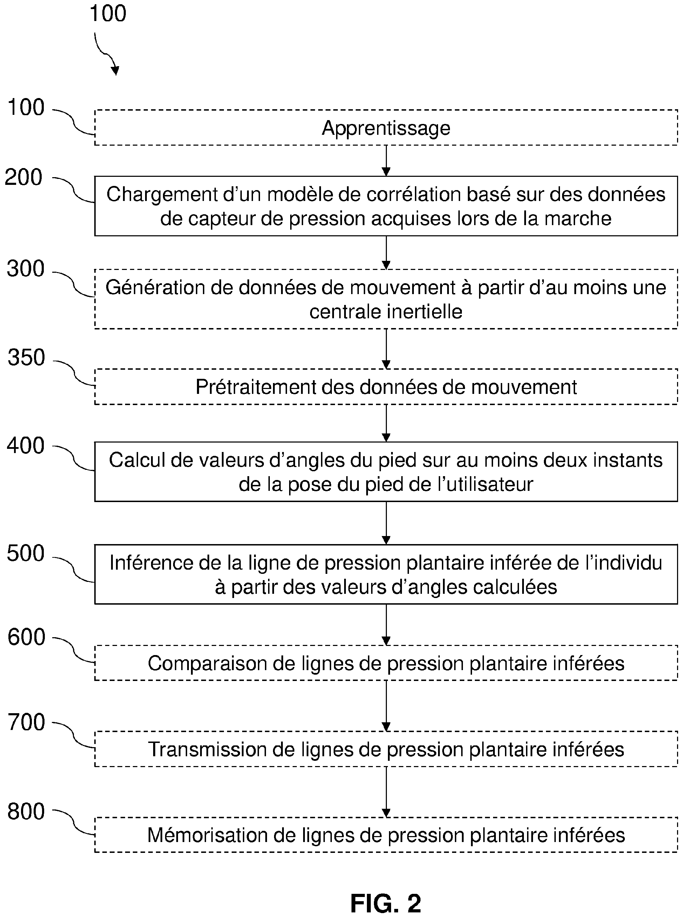

Comme cela sera détaillé par la suite et illustré à la figure 2, un procédé 1 d'inférence selon l'invention peut également comporter une étape d'apprentissage 100, une étape de génération 300 des données par la centrale inertielle, un prétraitement 350 des données générées par les capteurs de mouvement, une étape de comparaison 600 de lignes de pression plantaire 30 inférées, une transmission 700 de la ligne de pression plantaire inférée, ou encore une mémorisation 800 des données, notamment des données en lien avec la ligne de pression plantaire inférée.

Le procédé 1 d'inférence d'une ligne de pression plantaire 30 selon l'invention est de préférence mis en œuvre par un ou plusieurs microprocesseurs 22. Comme cela sera détaillé par la suite, le procédé est mis en œuvre à partir de données comportant des données de mouvement, ou calculées à partir de données de mouvement, générées par au moins une centrale inertielle 21 couplé à une chaussure portée par ledit individu.

Le ou les microprocesseurs 22 mettant en œuvre le procédé selon l'invention pourront être intégrés dans un dispositif électronique intégrant également la ou les centrales inertielles ou bien être intégrés dans un dispositif informatique, tel qu'un ordinateur ou un serveur informatique, configuré pour recevoir des données générées par la ou les centrales inertielles. Un dispositif d'inférence 2 selon l'invention sera détaillé plus avant dans la suite de cette description.

Comme illustré à la figure 2, un procédé selon l'invention peut comporter une étape d'apprentissage 100. Cette étape d'apprentissage est de préférence une étape réalisée au préalable de la mise en œuvre du procédé sur les données de mouvement d'un individu. En outre, elle peut être répétée et être enrichie de données générées dans le cadre de la présente invention.

Une étape d'apprentissage 100 pourra comporter une collecte des données d'entrainement puis une agrégation et un stockage de ces données cibles dans une base de données.

Les données d'entrainement comporteront des données provenant de capteurs de pression. En effet, la ligne de pression plantaire est calculée habituellement à partir de données de pression plantaire. Ces données de pression plantaire peuvent avoir été générées par n'importe quel dispositif connu d'une personne du métier et capable de générer des données de pression plantaire pour un individu. Par exemple, les données de pression plantaire pourront avoir été générées à partir de tapis roulant, ou encore de semelles intégrant des capteurs de pression. En outre, les données d'entrainement comporteront des données de mouvement du pied lors de phase d'appui, de préférence générées à partir d'une ou de plusieurs centrales inertielles.

L'étape d'apprentissage 100 permet la génération d'un modèle de corrélation basé sur des données de capteur de pression acquises lors de marches. En particulier, le modèle de corrélation définit, au travers d'une ou plusieurs instructions, une relation entre une pluralité de données obtenues à partir de capteurs de pression plantaire et une pluralité de valeurs d'angle de pied.

Un tel modèle est avantageusement configuré de façon à pouvoir inférer des données de position du centre de pression plantaire, et de préférence son déplacement, à partir de données de mouvement d'un pied.

De façon préférée, l'étape d'apprentissage 100 pourra comporter la génération de multiples modèles d'apprentissage automatique et une sélection du modèle d'apprentissage automatique fournissant les meilleures performances de prédiction.

Un procédé selon l'invention comporte une étape de chargement 200 d'un modèle de corrélation. Comme peut le comprendre la personne du métier, le chargement 200 peut correspondre à la mise en mémoire du modèle de corrélation pour son utilisation.

Cette étape implique que le modèle de corrélation a déjà été généré antérieurement, par exemple lors d'une étape d'apprentissage 100 et que c'est sa capacité d'inférence qui est utilisée dans le cadre du procédé selon l'invention.

De façon préférée, le modèle de corrélation correspond à un modèle d'apprentissage automatique. Par exemple, le modèle d'apprentissage automatique pourra être sélectionné parmi un modèle d'apprentissage automatique supervisé, non supervisé ou par renforcement.

Alternativement, il a été montré qu'un modèle de corrélation, n'étant pas dotée de capacité d'apprentissage automatique mais tout de même généré à partir de données de capteur de pression acquises lors de phase d'appui et des données de mouvement du pied correspondante, pouvait permettre d'inférer une ligne de pression plantaire.

Comme illustré à la figure 2, un procédé selon l'invention peut comporter une étape de génération 300 des données par au moins une centrale inertielle. La centrale inertielle 21 pourra par exemple être une centrale inertielle six axes ou neuf axes.

Dans le cadre de la génération 300 des données de mouvement, la ou les centrales inertielles 21 sont couplées à une chaussure. Par exemple, un procédé selon l'invention pourra utiliser des données de mouvement provenant de plusieurs centrales inertielles 21 couplées à une ou deux chaussures.

Comme cela sera détaillé par la suite, la centrale inertielle 21 générant les données de mouvement est de préférence positionnée dans un boitier électronique 20 agencé pour être intégré dans une semelle. Néanmoins, l'invention peut être mise en œuvre à partir de données générées par une ou plusieurs centrales inertielles positionnées sur une chaussure ou au niveau d'une cheville. En particulier, lors de l'étape de génération 300 des données par la centrale inertielle 21, la centrale inertielle 21 peut être positionnée dans un boitier électronique 20 fixé sur la chaussure portée par un individu.

Les données générées par la centrale inertielle 21 pourront de préférence être générées durant une marche de l'individu, une course ou tout autre exercice susceptible de générer des données mouvement pouvant être exploitée dans le cadre d'une analyse de cinétique de déplacement du pied dans l'espace. Ces données sont appelées des données de mouvement. La centrale inertielle est de préférence couplée à une chaussure mais de façon plus générale elle est couplée au pied d'un individu. Le couplage pouvant être direct ou par l'intermédiaire d'une chaussure.

Dans le cadre du procédé selon l'invention, les données de mouvement sont générées pour une pluralité d'instants de la pose du pied.

En particulier, lors de l'étape de génération 300 de données de mouvement par la ou les centrales inertielles, les données de mouvement sont générées pour au moins dix instants de la pose du pied, de préférence au moins vingt et de façon plus préférée au moins cinquante.

De façon préférée, les données de mouvement comporteront des données de mouvement générées entre l'instant de pose du talon et l'instant de pose des orteils. Les données de mouvement pourront également comporter des données de mouvement générées entre l'instant de décollage du talon et l'instant de décollage des orteils. En effet, la demanderesse a déterminé que ces données étaient d'une importance particulière pour améliorer la performance de prédiction du procédé selon l'invention.

En outre, le procédé 1 d'inférence d'une ligne de pression plantaire 30 inférée selon l'invention pourra comporter une étape de prétraitement 350 des données de mouvement générées par la ou les centrales inertielles.

En particulier, cette étape de prétraitement 350 des données de mouvement peut correspondre au prétraitement des valeurs d'accélération, de vitesse angulaire, et/ou de champs magnétique. Par exemple, elle peut comporter en particulier au moins un traitement sélectionné parmi : un filtrage fréquentiel, une suppression de la gravité sur les valeurs d'accélération, une suppression de la gravité, une suppression du bruit sur les valeurs d'accélération et une suppression de la dérive sur les valeurs de vitesse angulaire mesurées.

Ainsi, les données de mouvement peuvent correspondre à des données générées par une centrale inertielle 21 qui ont été normalisées, filtrées, complétées ou encore à des données ayant été fusionnées par exemple par un filtrage de Kalman. De façon préférée, les données de mouvement comportent des valeurs variables sous forme de séries temporelles. Ces valeurs variables pourront de préférence correspondre à des valeurs d'accélération et de vitesse angulaire. Elles pourront éventuellement comporter des données de champs magnétiques ou une fusion de ces données.

Comme illustré aux figures 1 et 2, un procédé selon l'invention comporte une étape de calcul 400 de valeurs d'angles du pied. En particulier, ce calcul 400 de valeurs d'angles du pied est fait par rapport au référentiel prédéterminé.

L'étape de calcul 400 de valeurs d'angles du pied correspond de façon préférée à une étape de calcul de valeurs d'angles pour les deux pieds de l'individu de façon à générer une ligne de pression plantaire 30 inférée pour chacun des pieds de l'individu.

Ces valeurs d'angles du pied sont en particulier calculées à partir des données de mouvement générées par l'au moins une centrale inertielle 21. En outre, de façon préférée, elles sont calculées sur au moins deux instants de la pose du pied de l'individu, ou phase d'appui.

De façon préférée, l'étape de calcul 400 de valeurs d'angles du pied comporte le calcul de valeurs d'angles pour au moins cinq instants de la pose du pied, de préférence au moins dix et de façon plus préférée au moins vingt.

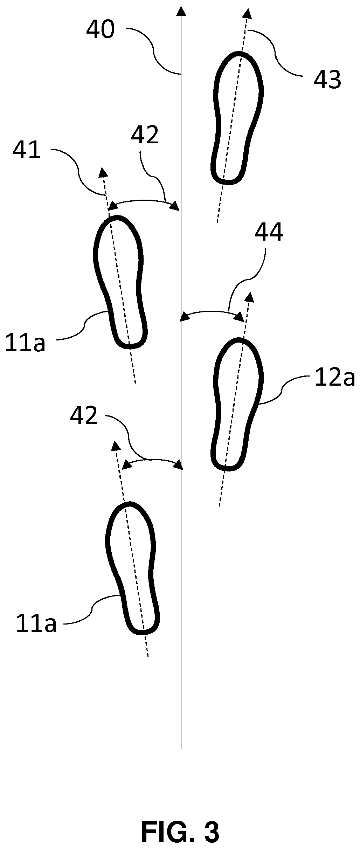

Il existe de nombreuses méthodes pour calculer des valeurs d'angles du pied en fonction des référentiels utilisés. En effet, les valeurs d'angles du pied correspondent généralement à des valeurs d'angle d'un pied par rapport à son environnement. Des exemples de valeurs d'angles du pied pouvant être calculés sont illustrés à la figure 3.

Ces valeurs d'angles 42,44 pourront par exemple être calculées en fonction de la ligne de marche 40 de l'individu. Elles pourront également être calculées par rapport au sol ou encore par rapport l'axe antéropostérieur 41,43 du pied 11a, 12a de l'individu. La ligne de marche peut être quant à elle calculée classiquement par les méthodes connues de la personne du métier puis utilisée comme référentiel pour le calcul des valeurs d'angles du pied dans le cadre de la présente invention.

De façon préférée, les valeurs d'angles du pied calculées comportent des valeurs d'angles sélectionnées parmi : un angle de l'axe antéro-postérieur du pied par rapport à sa ligne de progression, un angle de l'axe antéro-postérieur du pied par rapport à un plan formé par le sol, un angle de l'axe transversal du pied par rapport à sa ligne de progression ou un angle de l'axe transversal du pied par rapport à un plan formé par le sol.

Outre, le fait d'utiliser des valeurs d'angle du pied calculées en plusieurs instants de la pose du pied, la présente invention tire un avantage particulier de l'utilisation de valeurs de plusieurs angles du pied. Ainsi, de façon préférée, l'étape de calcul 400 de valeurs d'angles du pied comporte le calcul de valeurs d'au moins deux angles du pied, de préférence d'au moins trois angles du pied et de façon plus préférée au moins quatre angles du pied.

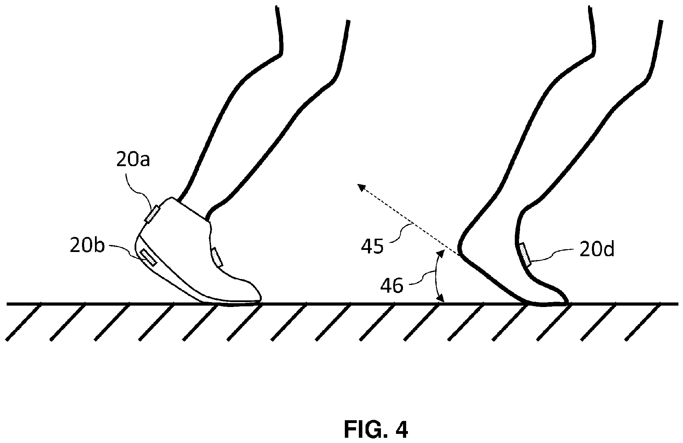

Par exemple, la figure 4 illustre un pied d'un individu couplé des centrales inertielles positionnées en trois emplacements différents : au niveau du contrefort 20a ; dans la semelle 20b extérieure ou intérieure ; ou encore sur le devant du pied 20c, par exemple au niveau des lacets ou de la languette.

La figure 4 montre également que l'invention peut être mise en œuvre à partir d'une centrale inertielle 20d directement couplée au pied de l'individu. Ce couplage peut par être utilisé une matière adhésive permettant de coller la centrale inertielle temporairement sur le pied ou encore grâce à un accessoire capable de maintenir la centrale inertielle contre le pied de l'individu. L'accessoire pourra par exemple être élastique et prendre la forme d'une chevillière ou d'un strap.

Parmi les angles pouvant être utilisés dans le cadre de l'invention, nous pouvons par exemple citer l'angle de frappe correspondant à une mesure de l'angle entre la base du pied et le sol au contact initial. Cet angle peut continuer à être mesure durant la phase d'attaque du pas jusqu'à la phase de pas antérieur. Comme illustré, l'angle 46 entre la base du pied 45 et le sol peut également être mesuré lors de la phase de propulsion.

Comme illustré aux figures 1 et 2, un procédé selon l'invention comporte une étape d'inférence 500 de la ligne de pression plantaire 30 de l'individu. Comme illustré à la figure 5, la ligne de pression plantaire 30 peut être considérée comme correspondant à une pluralité de centres de pression plantaire inférés.

L'étape d'inférence 500 pourra comporter la détermination d'une ligne de pression plantaire 30 pour chacun des pieds 11a,12a de l'individu.

Cette étape d'inférence 500 est de préférence réalisée à partir des valeurs d'angles calculées sur les au moins deux instants et du modèle de corrélation.

De façon préférée, l'étape d'inférence 500 comporte l'utilisation de valeurs d'angles calculées pour au moins cinq instants de la pose du pied, de préférence au moins dix et de façon plus préférée au moins vingt.

Avantageusement, les valeurs d'angles calculées utilisées lors de l'inférence 500 de la ligne de pression plantaire 30 comportent une majorité de valeurs d'angles calculées à partir de données de mouvement générées entre l'instant de pose du talon et l'instant de pose des orteils et entre l'instant de décollage du talon et l'instant de décollage des orteils.

De façon préférée, les valeurs d'angles calculées utilisées lors de l'inférence 500 de la ligne de pression plantaire 30 comportent au moins 60 % de valeurs d'angles calculées à partir de données de mouvement générée entre l'instant de pose du talon et l'instant de pose des orteils et entre l'instant de décollage du talon et l'instant de décollage des orteils.

De façon plus préférée, les valeurs d'angles calculées utilisées lors de l'inférence 500 de la ligne de pression plantaire 30 comportent au moins 70 % de valeurs d'angles calculées à partir de données de mouvement générée entre l'instant de pose du talon et l'instant de pose des orteils et entre l'instant de décollage du talon et l'instant de décollage des orteils.

De façon encore plus préférée, les valeurs d'angles calculées utilisées lors de l'inférence 500 de la ligne de pression plantaire 30 comportent au moins 80 % de valeurs d'angles calculées à partir de données de mouvement générée entre l'instant de pose du talon et l'instant de pose des orteils et entre l'instant de décollage du talon et l'instant de décollage des orteils.

Cette étape d'inférence 500 est de préférence réalisé à partir de valeurs de plusieurs angles. De façon préférée, l'étape d'inférence 500 est de préférence réalisée à partir des valeurs d'au moins deux angles du pied, de préférence d'au moins trois angles du pied et de façon plus préférée au moins quatre angles du pied.

Dans le cadre de l'invention, l'étape d'inférence 500 pourra être répétée de façon à établir une ligne de pression plantaire 30 inférée à partir de plusieurs phases d'appui, par exemple obtenues lors de cycles de marche ou de course. Cette ligne de pression pourra par exemple être composée à partir d'un calcul prenant en compte plusieurs répétitions tel qu'un calcul de moyenne ou de médiane.

Comme cela a été abordée, un des avantages de la présente invention est de pouvoir prédire une ligne de pression plantaire sans avoir à utiliser de capteur de pression alors que cela constituait avant la présente invention un passage obligé de la podométrie lors de l'établissement d'une ligne de pression plantaire. Ainsi, avantageusement, la détermination de la ligne de pression plantaire 30 inférée ne prend pas en compte de données provenant d'un capteur de pression couplé à la chaussure de l'individu. De façon plus large, l'inférence de la ligne de pression plantaire 30 selon l'invention ne prend pas en compte de données provenant d'un capteur de pression ayant généré des données de pression associées audit individu.

Comme illustré à la figure 2, un procédé conforme à l'invention peut comporter une étape de comparaison 600 de lignes de pression plantaire 30 inférées.

En particulier, le procédé selon l'invention pourra comporter une comparaison des lignes de pression plantaire 30 inférées de chacun des pieds de l'individu.

Il pourra également comporter une comparaison des lignes de pression plantaire 30 inférées pour un même pied pour deux intervalles temporels différents.

Comme illustré à la figure 2, un procédé conforme à l'invention peut comporter une étape de transmission 700 de la ligne de pression plantaire 30 à un dispositif électronique distant 50, 60, également dénommé terminal externe 50, 60.

De préférence, le procédé peut également comporter une étape de mémorisation 800 de données. Cette mémorisation se fait de préférence en continu. En particulier cela peut correspondre à la mémorisation de l'ensemble des données générées et/ou calculées dans le cadre d'un procédé selon l'invention. Les données mémorisées peuvent par exemple être des données brutes telles qu'elles ont été générées par les capteurs de mouvement, des données prétraitées, des données transformées ou encore des données de lignes de pressions plantaires inférées. De préférence, les données mémorisées correspondent à des données de lignes de pressions plantaires inférées.

Avantageusement, le calcul d'une ligne de pressions plantaires 30 inférée est fait en temps réel, c'est-à-dire moins de 1 heure après la génération des données par l'un ou plusieurs des capteurs de mouvement d'une semelle, de préférence moins de 10 minutes, de façon plus préférée moins d'une minute, de façon encore plus préférée moins de 10 secondes.

Selon un autre aspect, l'invention porte sur un dispositif d'inférence 2 d'une ligne de pression plantaire 30. Comme cela a été décrit, la ligne de pression plantaire 30 comporte une pluralité de centres de pression inférés pour un pied d'un individu.

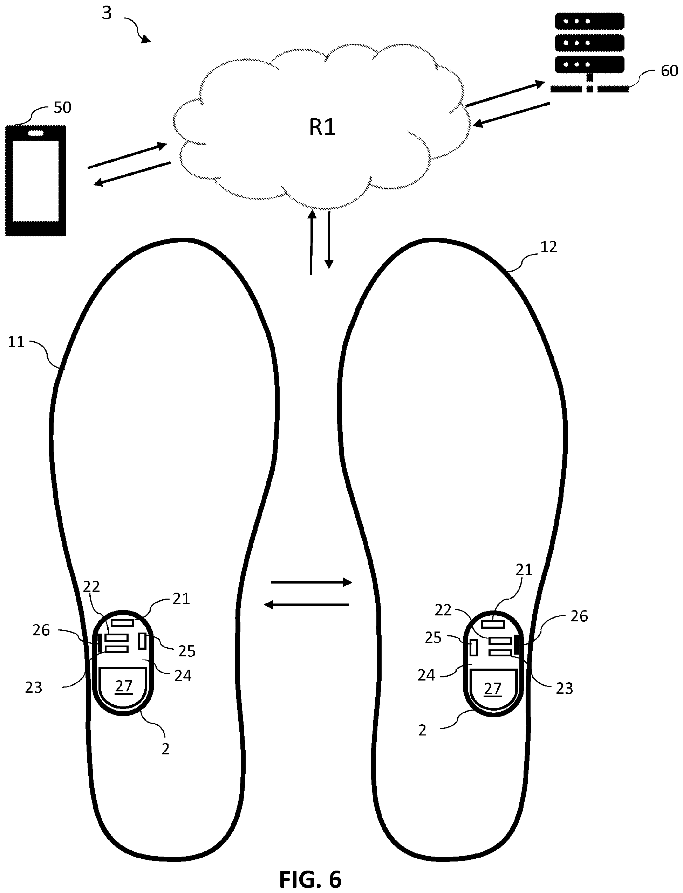

Un dispositif d'inférence 2 selon l'invention est détaillé dans la figure 6. Un tel dispositif d'inférence 2 peut en particulier être utilisé pour inférer une ligne de pression plantaire 30 à partir de données de mouvement générées par une ou plusieurs centrales inertielles 21. Il s'applique lorsque les capteurs de mouvement 21 sont couplé au pied d'un individu par exemple couplés à la chaussure d'un individu.

De façon préférée, un dispositif d'inférence 2 comporte un ou plusieurs microprocesseurs 22 configurés pour exécuter un procédé selon l'invention, de préférence le procédé d'inférence 1, et ses différents modes de réalisation, préférés, avantageux ou non.

En particulier, les un ou plusieurs microprocesseurs 22 sont configurés de façon à charger un modèle de corrélation. De façon préférée, le modèle de corrélation définie une relation entre une pluralité de données obtenues à partir de capteurs de pression plantaire et une pluralité de valeurs d'angle de pied par rapport à un référentiel prédéterminé.

Les un ou plusieurs microprocesseurs 22 peuvent aussi être configurés de façon à calculer des valeurs d'angles du pied par rapport au référentiel prédéterminé, à partir de données de mouvement générées par au moins une centrale inertielle 21, sur au moins deux instants de la phase d'appui du pied dudit l'individu.

En outre, les un ou plusieurs microprocesseurs 22 peuvent aussi être configurés de façon à inférer une pluralité de centres de pression pour former la ligne de pression plantaire 30 du pied de l'individu, à partir des valeurs d'angles calculées sur les au moins deux instants de la phase d'appui du pied et du modèle de corrélation.

Le dispositif d'inférence 2 d'une ligne de pression plantaire 30 pourra également comporter la ou les centrales inertielles 21. Dans ce cas, il sera de préférence de taille réduite et pourra être fixé au pied d'un individu par tout moyen. Il pourra par exemple être directement fixé au pied de façon amovible grâce à un dispositif adhérent. Il pourra également être couplé au pied de l'individu par l'intermédiaire d'une chaussure portée par l'individu. Dans ce cas, il pourra être fixé à la chaussure ou intégré à la semelle. De façon préférée, le dispositif d'inférence 2 est intégré dans une semelle, par exemple de façon amovible.

Avantageusement, le dispositif d'inférence 2 pèse moins de 10 grammes et est agencé de façon à pouvoir se loger dans une semelle intérieure et/ou extérieure. Un faible volume, par exemple inférieur à 10 cm$^3$, limite l'impact sur le confort de l'individu et présente l'avantage d'optimiser les coûts de production en rendant moins onéreux et plus simple l'intégration de cette technologie dans la semelle lors du processus industriel.

En particulier, un tel dispositif d'inférence 2 selon l'invention comporte un capteur de mouvement tel qu'une centrale inertielle 21 configurée pour générer des données de mouvement d'un pied de l'individu. Au cours de l'utilisation du dispositif d'inférence 2 par un individu la centrale inertielle 21 acquière des signaux représentatifs d'un paramètre de mouvement (accélération et/ou vitesse, par exemple vitesse angulaire) du pied de l'utilisateur, selon les axes X, Y, Z.

La centrale inertielle 21 est par exemple constituée d'au moins un accéléromètre et un gyroscope. De façon préférée, elle comporte plusieurs accéléromètres et gyroscopes. Le dispositif d'inférence 2 peut également comporter un ou plusieurs magnétomètres de façon à acquérir trois signaux bruts supplémentaires correspondant aux valeurs de champs magnétiques sur trois dimensions. Ainsi, les données de mouvement peuvent être traitées pour générer des données de mouvement issu d'une fusion entre des données inertielles et magnétiques.

Chaque dispositif d'inférence 2 peut comporter par ailleurs d'autres capteurs, notamment un inclinomètre, un baromètre, un capteur de température et un altimètre pour bénéficier d'une précision accrue.

Le dispositif d'inférence 2 selon l'invention comporte également une mémoire de données 23, configurée pour mémoriser au moins une partie des données de mouvement générées et/ou des lignes de pression plantaire inférée. La mémoire de données 23 est également configurée pour stocker le modèle de corrélation.

Les différents composants du dispositif d'inférence 2 sont de préférence agencés sur une carte électronique 24 (ou circuit imprimé), mais l'invention peut prévoir divers types d'agencement comme par exemple un seul module cumulant l'ensemble des fonctions décrites ici. De même, ces moyens peuvent être divisés en plusieurs cartes électroniques ou bien rassemblés sur une seule carte électronique. En outre, lorsque l'on prête une action à un dispositif, un moyen ou un module, celle-ci est en fait généralement effectuée par un microprocesseur du dispositif ou module commandé par des codes instructions enregistrés dans une mémoire. De même, si l'on prête une action à une application, celle-ci est en fait effectuée par un microprocesseur du dispositif dans une mémoire duquel les codes instructions correspondant à l'application sont enregistrés. Lorsqu'un dispositif ou module émet ou reçoit un message, ce message est émis ou reçu par une interface de communication.

Le dispositif d'inférence 2 selon l'invention comporte avantageusement un ou plusieurs moyens de communication 25. Les un ou plusieurs moyens de communication 25 sont aptes à recevoir et à transmettre les données sur au moins un réseau de communication R1. De préférence la communication est opérée par l'intermédiaire d'un protocole sans fil tel que wifi, 3G, 4G, 5G et/ou Bluetooth. De préférence le protocole de communication est un protocole BLE ou ANT+. Ces protocoles de communication permettent une consommation faible en énergie.

De préférence, le dispositif d'inférence 2 selon l'invention comporte un premier moyen de communication 25 configuré de façon à ce que le dispositif d'inférence 2 soit apte à transmettre au moins une ligne de pression plantaire 30 inférée à un terminal externe 50,60. Ces données peuvent être transmises en temps réel ou en différé à un terminal externe 50,60. Le terminal externe ou le dispositif électronique distant 50,60 peut par exemple être un système distant tel qu'une tablette, un téléphone mobile (« smartphone » en terminologie anglosaxonne), un ordinateur ou un serveur.

Avantageusement, un dispositif d'inférence 2 selon l'invention comporte en outre un second moyen de communication 25 configuré de façon à ce qu'un premier dispositif d'inférence 2 soit apte à communiquer avec un second dispositif d'inférence 2.

En particulier, les dispositifs d'inférence 2 peuvent être configurés pour communiquer entre eux et pour initier la collecte de données de mouvement et l'inférence de ligne de pression plantaire d'un individu seulement après réception d'un message de l'autre dispositif d'inférence 2. Cela participe à la synchronisation des dispositifs d'inférence 2.

Avantageusement, si un dispositif d'inférence 2 venait à se déconnecter ou perdre la synchronisation dans le temps par rapport à l'autre dispositif d'inférence 2, un procédé selon l'invention pourrait comprendre une étape de synchronisation des dispositifs d'inférence 2. Ainsi, un signal de recherche est envoyé par le dispositif d'inférence 2 connecté, le dispositif d'inférence 2 déconnecté reçoit le signal de recherche et se synchronise sur le dispositif d'inférence 2 connecté.

En outre, le dispositif d'inférence 2 selon l'invention peut comporter un port pour connexion filaire 26, de préférence protégé par une languette amovible. Ce port pour connexion filaire peut être par exemple un port USB ou firewire. Avantageusement, le port USB est également résistant à l'eau ou l'humidité. En outre, le port USB est avantageusement surmonté d'une poutrelle en polymère permettant de lui conférer une plus grande résistance en condition d'utilisation. Ce port pour connexion filaire 26 peut être utilisé comme évoqué ci-dessus pour recharger la batterie mais également pour échanger des données et par exemple mettre à jour le micrologiciel de la carte électronique portant les différents composants du dispositif d'inférence 2.

De préférence, la languette amovible ou USB cover, permet de protéger le port USB de corps étrangers. Par exemple, la languette amovible permet de protéger le port USB de l'eau ou de la poussière. Une telle languette peut de préférence être réalisée en polymère de type élastomère ou polyuréthane.

En outre, le dispositif d'inférence 2 selon l'invention peut comporter une source d'énergie 27. La source d'énergie est de préférence de type batterie, rechargeable ou non. De préférence la source d'énergie est une batterie rechargeable. En outre, elle peut être associée à un système de recharge par le mouvement ou par une énergie extérieure. Le système de recharge par une énergie extérieure peut notamment être un système de recharge par connexion filaire, un système de recharge par induction ou encore photovoltaïque.

La source d'énergie 27 est de préférence de type batterie, rechargeable ou non. De préférence la source d'énergie est une batterie rechargeable. La recharge peut être réalisée selon différentes technologies telles que : par chargeur, avec un connecteur affleurant au niveau de la semelle ; avec un dispositif de recharge mécanique intégré à la semelle, comme par exemple un dispositif piézoélectrique apte à fournir une énergie électrique à partir de la marche ; avec un dispositif sans contact, par exemple par induction ; et/ou avec un dispositif photovoltaïque.

En outre, selon un autre aspect, la présente invention porte sur un système d'inférence 3 d'une ligne de pression plantaire 30 d'un individu.

Un tel système d'inférence 3 d'une ligne de pression plantaire 30 d'un individu, décrit en lien avec la figure 6, comportera avantageusement un ou plusieurs dispositifs d'inférence 2 selon la présente invention. En particulier, le système d'inférence 3 selon l'invention comporte deux dispositifs d'inférence 2 selon la présente invention.

En particulier, chacun des dispositifs d'inférence 2 est conçu de manière à pouvoir communiquer indépendamment avec l'autre et/ou directement avec un terminal externe 50,60 afin de pouvoir échanger ses propres informations sur la ligne de pression plantaire 30 inférée.

En particulier, le système d'inférence 3 selon l'invention peut comporter une paire de semelles 11,12 comportant chacune un dispositif d'inférence 2 selon l'invention. Les semelles 11, 12 utilisables dans le cadre du système d'inférence 3 selon l'invention peuvent par exemple correspondre à des semelles extérieures ou à des semelles intérieures, de chaussures. Ces semelles peuvent être amovibles ou être intégrées de manière permanente au semelage des chaussures. Classiquement, les semelles 11, 12 composants ladite paire de semelles, comportent chacune un dispositif d'inférence 2 selon l'invention. Comme présenté sur la figure 6, le dispositif d'inférence 2 est de préférence positionné au niveau d'une portion médiane de la semelle.

Comme mentionné, le système d'inférence 3 selon l'invention peut comporter un terminal externe 50,60 configuré pour recevoir des données provenant du ou des dispositif(s) d'inférence 2.

Avantageusement, une application dédiée est installée sur ce terminal externe 50, 60 afin de traiter les informations transmises par le ou les dispositif(s) d'inférence 2 et permettre à l'utilisateur d'interagir avec l'invention. Il est alors par exemple possible d'accéder au terminal externe 50, 60 via une interface web. Toutes les communications avec le terminal externe 50, 60 peuvent être sécurisées par exemple par des protocoles HTTPS (pour « HyperText Transfer Protocol Secure » selon une terminologie anglo-saxonne) et cryptage AES (pour « Advanced Encryption Standard » selon une terminologie anglo-saxonne) 512. Ainsi, cela peut permettre, via un client, l'accès aux données par du personnel médical en charge du suivi de l'utilisateur.

Le terminal externe 50,60 est généralement une tablette, un téléphone mobile (« smartphone » en terminologie anglosaxonne), une passerelle, un routeur, un ordinateur ou un serveur. Il peut être apte à transférer ces données à un serveur distant. Il est alors par exemple possible d'accéder à ce serveur distant via une interface web.

Ainsi, l'utilisateur peut accéder à des données liées à la ou aux lignes de pression plantaires 30 inférées.

L'invention peut faire l'objet de nombreuses variantes et applications autres que celles décrites ci-dessus. En particulier, sauf indication contraire, les différentes caractéristiques structurelles et fonctionnelles de chacune des mises en œuvre décrite ci-dessus ne doivent pas être considérées comme combinées et/ou étroitement et/ou inextricablement liées les unes aux autres, mais au contraire comme de simples juxtapositions. En outre, les caractéristiques structurelles et/ou fonctionnelles des différents modes de réalisation décrits ci-dessus peuvent faire l'objet en tout ou partie de toute juxtaposition différente ou de toute combinaison différente.

**Revendications**

**1.** Procédé (1) d'inférence d'une ligne de pression plantaire (30), ladite la ligne de pression plantaire (30) correspondant à l'évolution temporelle de la position du barycentre des pressions plantaires lors du déplacement d'un individu et comportant une pluralité de positions successives dudit barycentre des pressions plantaires pour un pied dudit individu, ledit procédé étant mis en œuvre par un ou plusieurs microprocesseurs (22) à partir de données de mouvement générées par au moins une centrale inertielle (21) couplée à une chaussure portée par ledit individu ou au pied dudit individu, ladite au moins une centrale inertielle (21) étant configurée pour générer des données de mouvement pour une pluralité d'instants d'une phase d'appui d'un pied dudit l'individu, ledit procédé comportant les étapes suivantes :

- Chargement (200) d'un modèle de corrélation, ledit modèle de corrélation définissant une relation entre une pluralité de données obtenues à partir de capteurs de pression plantaire et une pluralité de valeurs d'angle de pied par rapport à un référentiel prédéterminé, ladite relation permettant de déterminer la position du barycentre des pressions plantaires à chaque instant d'une phase d'appui ;
- Calcul (400) de valeurs d'angles du pied par rapport au référentiel prédéterminé, à partir des données de mouvement générées par l'au moins une centrale inertielle (21), sur au moins deux instants de la phase d'appui du pied dudit l'individu ; et
- Inférence (500) d'une pluralité de positions successives du barycentre des pressions plantaires pour former la ligne de pression plantaire (30) du pied dudit individu, à partir des valeurs d'angles calculées sur les au moins deux instants de la phase d'appui du pied et du modèle de corrélation.

**2.** Procédé (1) d'inférence d'une ligne de pression plantaire (30) selon la revendication 1, **caractérisé en ce que** l'inférence (500) est répétée de façon à établir la ligne de pression plantaire (30) inférée à partir de plusieurs phases d'appui.

**3.** Procédé (1) d'inférence d'une ligne de pression plantaire (30) selon l'une quelconque des revendications 1 ou 2, **caractérisé en ce que** l'étape de calcul (400) est réalisée à partir de données de mouvement générées par les centrales inertielles (21) alors qu'elles sont positionnées dans une semelle amovible ou non amovible.

**4.** Procédé (1) d'inférence d'une ligne de pression plantaire (30) selon l'une quelconque des revendications 1 à 3, **caractérisé en ce que** l'étape de calcul (400) est réalisée à partir de données de mouvement générées par les centrales inertielles (21) alors qu'elles sont positionnées dans un boitier électronique agencé pour être fixé sur la chaussure portée par ledit individu.

**5.** Procédé (1) d'inférence d'une ligne de pression plantaire (30) selon l'une quelconque des revendications 1 à 3, **caractérisé en ce que** l'étape de calcul (400) est réalisée à partir de données de mouvement générées par les centrales inertielles (21) alors qu'elles sont positionnées dans un boitier électronique, ledit boitier électronique comportant également l'un ou plusieurs microprocesseurs (22) configurés pour calculer les valeurs d'angles du pied par rapport au référentiel prédéterminé.

**6.** Procédé (1) d'inférence d'une ligne de pression plantaire (30) selon la revendication précédente, **caractérisé en ce que** les calcul (400) de valeurs d'angles du pied et inférence (500) d'une pluralité de centres de pression sont mis en œuvre par les un ou plusieurs microprocesseurs (22) positionnés dans un boitier électronique intégré à une semelle.

**7.** Procédé (1) d'inférence d'une ligne de pression plantaire (30) selon l'une quelconque des revendications 1 ou 2, **caractérisé en ce que** l'étape de calcul (400) est réalisée à partir de données de mouvement générées par les centrales inertielles (21) alors qu'elles sont positionnées en trois emplacements différents du pied dudit individu.

**8.** Procédé (1) d'inférence d'une ligne de pression plantaire (30) selon l'une quelconque des revendications précédentes, **caractérisé en ce que** le calcul (400) de valeurs d'angles du pied comporte le calcul de valeurs d'au moins deux angles du pied.

**9.** Procédé (1) d'inférence d'une ligne de pression plantaire (30) selon l'une quelconque des revendications précédentes, dans lequel ledit référentiel prédéterminé est externe au corps humain externe au corps humain et sélectionné parmi : le sol, une ligne de marche calculée ou une trajectoire calculée dudit pied dudit individu.

**10.** Procédé (1) d'inférence d'une ligne de pression plantaire (30) selon la revendication précédente, **caractérisé en ce que** les valeurs d'angles du pied calculées, par rapport au référentiel prédéterminé, comportent des valeurs d'angles sélectionnées parmi : un angle de l'axe antéro-postérieur du pied par rapport à sa ligne de progression, un angle de l'axe antéro-postérieur du pied par rapport au sol, un angle de l'axe transversal du pied par rapport à sa ligne de progression, un angle de l'axe transversal du pied par rapport au sol ou l'angle de frappe entre la base du pied et le sol au contact initial.

**11.** Procédé (1) d'inférence d'une ligne de pression plantaire (30) selon l'une quelconque des revendications précédentes, **caractérisé en ce qu'**il comporte une comparaison des lignes de pressions plantaires (30) de chacun des pieds de l'individu.

**12.** Procédé (1) d'inférence d'une ligne de pression plantaire (30) selon l'une quelconque des revendications précédentes, **caractérisé en ce qu'**il comporte une comparaison des lignes de pressions plantaires (30) obtenues pour un même pied pour deux intervalles temporels différents.

**13.** Procédé (1) d'inférence d'une ligne de pression plantaire (30) selon l'une quelconque des revendications précédentes, **caractérisé en ce qu'**il comporte une étape de prétraitement des données de mouvement générées par l'au moins une centrale inertielle (21), l'étape de prétraitement comportant un filtrage fréquentiel, une suppression de la gravité, et/ou une suppression de la dérive.

**14.** Dispositif d'inférence (2) d'une ligne de pression plantaire (30), ladite ligne de pression plantaire (30) correspondant à l'évolution temporelle de la position du barycentre des pressions plantaires lors du déplacement d'un individu et comportant une pluralité de positions successives dudit barycentre des pressions plantaires pour un pied dudit individu, ledit dispositif d'inférence (2) comportant un ou plusieurs microprocesseurs (22), lesdits un ou plusieurs microprocesseurs (22) étant configuré pour :

- Charger un modèle de corrélation, ledit modèle de corrélation définissant une relation entre une pluralité de données obtenues à partir de capteurs de pression plantaire et une pluralité de valeurs d'angle de pied par rapport à un référentiel prédéterminé, ladite relation permettant de déterminer la position du barycentre des pressions plantaires à chaque instant d'une phase d'appui ;
- Calculer des valeurs d'angles du pied par rapport au référentiel prédéterminé, à partir de données de mouvement générées par au moins une centrale inertielle (21), sur au moins deux instants de la phase d'appui du pied dudit l'individu ; et
- Inférer une pluralité de positions successives du barycentre des pressions plantaires pour former la ligne de pression plantaire (30) du pied dudit individu, à partir des valeurs d'angles calculées sur les au moins deux instants de la phase d'appui du pied et du modèle de corrélation.

**15.** Dispositif d'inférence (2) d'une ligne de pression plantaire (30) selon la revendication précédente, **caractérisé en ce qu'**il comporte en outre, l'au moins une centrale inertielle (21) couplée à une chaussure portée par ledit individu ou au pied dudit individu, ladite au moins une centrale inertielle (21) étant configurée pour générer des données de mouvement pour une pluralité d'instants d'une phase d'appui d'un pied dudit l'individu.

**Patentansprüche**

**1.** Verfahren (1) zur Ermittlung einer Plantardrucklinie (30), wobei die genannte Plantardrucklinie (30) der zeitlichen Entwicklung der Position des Schwerpunkts der Plantardrücke während der Fortbewegung einer Person entspricht und eine Vielzahl aufeinanderfolgender Positionen des genannten Schwerpunkts der Plantardrücke für einen Fuß der genannten Person aufweist, wobei das genannte Verfahren durch einen oder mehrere Mikroprozessoren (22) aus Bewegungsdaten implementiert wird, die durch mindestens eine inertiale Messeinheit (21) erzeugt werden, die an einem von der genannten Person getragenen Schuh oder am Fuß der genannten Person gekoppelt ist, wobei die genannte mindestens eine inertiale Messeinheit (21) konfiguriert ist, Bewegungsdaten für mehrere Zeitpunkte einer Standphase eines Fußes der genannten Person zu erzeugen, und wobei das genannte Verfahren folgende Schritte umfasst:

• Laden (200) eines Korrelationsmodells, wobei das genannte Korrelationsmodell eine Beziehung zwischen einer Vielzahl von Daten aus Plantardrucksensoren und einer Vielzahl von Fußwinkelwerten relativ zu einem vorbestimmten Referenzrahmen definiert, wobei die genannte Beziehung die Bestimmung der Position des Schwerpunkts der Plantardrücke zu jedem Zeitpunkt einer Standphase ermöglicht;
• Berechnung (400) von Fußwinkelwerten relativ zum vorbestimmten Referenzrahmen aus den von der genannten mindestens einen inertialen Messeinheit (21) erzeugten Bewegungsdaten zu mindestens zwei Zeitpunkten der Standphase des Fußes der genannten Person; und
• Ermittlung (500) einer Vielzahl aufeinanderfolgender Positionen des Schwerpunkts der Plantardrücke zur Bildung der Plantardrucklinie (30) des Fußes der genannten Person aus den berechneten Winkelwerten über die genannten mindestens zwei Zeitpunkte der Standphase und dem Korrelationsmodell.

**2.** Verfahren (1) zur Ermittlung einer Plantardrucklinie (30) nach Anspruch 1, **dadurch gekennzeichnet, dass** der Inferenzschritt (500) wiederholt wird, um die aus mehreren Standphasen abgeleitete Plantardrucklinie (30) zu erstellen.

3. Verfahren (1) zur Ermittlung einer Plantardrucklinie (30) nach einem der Ansprüche 1 oder 2, **dadurch gekennzeichnet, dass** der Berechnungsschritt (400) auf Grundlage von Bewegungsdaten durchgeführt wird, die von den inertialen Messeinheiten (21) erzeugt werden, während diese in einer herausnehmbaren oder nicht herausnehmbaren Sohle positioniert sind.

4. Verfahren (1) zur Ermittlung einer Plantardrucklinie (30) nach einem der Ansprüche 1 bis 3, **dadurch gekennzeichnet, dass** der Berechnungsschritt (400) auf Grundlage von Bewegungsdaten durchgeführt wird, die von den inertialen Messeinheiten (21) erzeugt werden, während diese in einem zur Befestigung an dem von der genannten Person getragenen Schuh vorgesehenen Gehäuse angeordnet sind.

5. Verfahren (1) zur Ermittlung einer Plantardrucklinie (30) nach einem der Ansprüche 1 bis 3, **dadurch gekennzeichnet, dass** der Berechnungsschritt (400) auf Grundlage von Bewegungsdaten durchgeführt wird, die von den inertialen Messeinheiten (21) erzeugt werden, während diese in einem elektronischen Gehäuse positioniert sind, wobei das genannte elektronische Gehäuse ferner den oder die Mikroprozessor(en) (22) enthält, die zur Berechnung der Fußwinkelwerte relativ zum vorbestimmten Referenzrahmen konfiguriert sind.

6. Verfahren (1) zur Ermittlung einer Plantardrucklinie (30) nach dem vorangehenden Anspruch, **dadurch gekennzeichnet, dass** die Berechnung (400) der Fußwinkelwerte und die Inferenz (500) einer Vielzahl von Druckschwerpunkten von dem oder den in einer in eine Sohle integrierten elektronischen Gehäuse angeordneten Mikroprozessor(en) (22) durchgeführt werden.

7. Verfahren (1) zur Ermittlung einer Plantardrucklinie (30) nach einem der Ansprüche 1 oder 2, **dadurch gekennzeichnet, dass** der Berechnungsschritt (400) auf Grundlage von Bewegungsdaten durchgeführt wird, die von den inertialen Messeinheiten (21) erzeugt werden, während diese an drei verschiedenen Positionen des Fußes der genannten Person angeordnet sind.

8. Verfahren (1) zur Ermittlung einer Plantardrucklinie (30) nach einem der vorangehenden Ansprüche, **dadurch gekennzeichnet, dass** die Berechnung (400) der Fußwinkelwerte die Ermittlung von Werten von mindestens zwei Fußwinkeln umfasst.

9. Verfahren (1) zur Ermittlung einer Plantardrucklinie (30) nach einem der vorangehenden Ansprüche, **dadurch gekennzeichnet, dass** der genannte vorbestimmte Referenzrahmen außerhalb des menschlichen Körpers liegt und ausgewählt wird aus: dem Boden, einer berechneten Ganglinie oder einer berechneten Trajektorie des genannten Fußes der genannten Person.

10. Verfahren (1) zur Ermittlung einer Plantardrucklinie (30) nach dem vorangehenden Anspruch, **dadurch gekennzeichnet, dass** die berechneten Fußwinkelwerte relativ zum vorbestimmten Referenzrahmen Winkelwerte ausgewählter Winkel umfassen aus:

einem Winkel der anteroposterioren Achse des Fußes relativ zu dessen Verlaufslinie, einem Winkel der anteroposterioren Achse des Fußes relativ zum Boden, einem Winkel der transversalen Achse des Fußes relativ zu dessen Verlaufslinie, einem Winkel der transversalen Achse des Fußes relativ zum Boden oder dem Auftreffwinkel zwischen der Fußsohle und dem Boden beim Erstkontakt.

11. Verfahren (1) zur Ermittlung einer Plantardrucklinie (30) nach den vorangehenden Ansprüchen, **dadurch gekennzeichnet, dass** ein Vergleich der Plantardrucklinien (30) beider Füße der Person vorgenommen wird.

12. Verfahren (1) zur Ermittlung einer Plantardrucklinie (30) nach den vorangehenden Ansprüchen, **dadurch gekennzeichnet, dass** ein Vergleich der für denselben Fuß für zwei unterschiedliche Zeitintervalle erhaltenen Plantardrucklinien (30) vorgenommen wird.

13. Verfahren (1) zur Ermittlung einer Plantardrucklinie (30) nach den vorangehenden Ansprüchen, **dadurch gekennzeichnet, dass** ein Schritt der Vorverarbeitung der von der genannten mindestens einen inertialen Messeinheit (21) generierten Bewegungsdaten umfasst wird, wobei der Vorverarbeitungsschritt eine Frequenzfilterung, eine Schwerkraftunterdrückung und/oder eine Driftunterdrückung umfasst.

14. Vorrichtung (2) zur Inferenz einer Plantardrucklinie (30), wobei die genannte Plantardrucklinie (30) der zeitlichen Entwicklung der Position des Schwerpunkts der Plantardrücke während der Fortbewegung einer Person entspricht und eine Vielzahl aufeinanderfolgender Positionen des genannten Schwerpunkts der Plantardrücke für einen Fuß der genannten Person umfasst, wobei die genannte Inferenzvorrichtung (2) einen oder mehrere Mikroprozessoren (22) umfasst, wobei der oder die genannten Mikroprozessor(en) (22) konfiguriert sind zu:

• Laden eines Korrelationsmodells, wobei das genannte Korrelationsmodell eine Beziehung zwischen einer Vielzahl von Daten aus Plantardrucksensoren und einer Vielzahl von Fußwinkelwerten relativ zu einem vorbestimmten Referenzrahmen definiert, wobei die genannte Beziehung die Bestimmung der Position des Schwerpunkts der Plantardrücke zu jedem Zeitpunkt einer Standphase ermöglicht;

• Berechnen von Fußwinkelwerten relativ zum vorbestimmten Referenzrahmen aus den von mindestens einer inertialen Messeinheit (21) erzeugten Bewegungsdaten über mindestens zwei Zeitpunkte der Standphase des Fußes der genannten Person; und

• Inferieren einer Vielzahl aufeinanderfolgender Positionen des Schwerpunkts der Plantardrücke zur Bildung der Plantardrucklinie (30) des Fußes der genannten Person aus den über die genannten mindestens zwei Zeitpunkte der Standphase berechneten Winkelwerten und dem Korrelationsmodell.

**15.** Vorrichtung (2) zur Inferenz einer Plantardrucklinie (30) nach dem vorangehenden Anspruch, **dadurch gekennzeichnet, dass** sie ferner mindestens eine inertiale Messeinheit (21) umfasst, die an einem von der genannten Person getragenen Schuh oder am Fuß der genannten Person gekoppelt ist, wobei die genannte mindestens eine inertiale Messeinheit (21) konfiguriert ist, Bewegungsdaten für eine Vielzahl von Zeitpunkten einer Standphase eines Fußes der genannten Person zu erzeugen.

**Claims**

**1.** A method (1) for inferring a plantar pressure line (30), said plantar pressure line (30) corresponding to the time evolution of the position of the barycenter of plantar pressures during the movement of an individual and including a plurality of successive positions of said barycenter of plantar pressures for a foot of said individual, said method being implemented by one or several microprocessors (22) from movement data generated by at least one inertial unit (21) coupled to a shoe worn by said individual or said individual's foot, said at least one inertial unit (21) being configured to generate movement data for a plurality of instants of a stance phase of a foot of said individual, said method including the following steps:

- loading (200) a correlation model, said correlation model defining a relationship between a plurality of data obtained from plantar pressure sensors and a plurality of foot angle values relative to a predetermined reference frame, said relationship enabling the determination of the position of the barycenter of plantar pressures at each instant of a stance phase;

- calculating (400) foot angle values relative to the predetermined reference frame, from the movement data generated by the at least one inertial unit (21), over at least two instants of the stance phase of the foot of said individual; and

- inferring (500) a plurality of successive positions of the barycenter of plantar pressures to form the plantar pressure line (30) of the said individual's foot, from the angle values calculated over the at least two instants of the stance phase of the foot and from the correlation model.

**2.** The method (1) for inferring a plantar pressure line (30) according to claim 1, **characterized in that** the inference step (500) is repeated so as to establish the plantar pressure line (30) inferred from several stance phases.

**3.** The method (1) for inferring a plantar pressure line (30) according to any one of claims 1 or 2, **characterized in that** the calculation step (400) is carried out from movement data generated by the inertial units (21) while they are positioned in a removable or non-removable sole.

**4.** The method (1) for inferring a plantar pressure line (30) according to any one of claims 1 to 3, **characterized in that** the calculation step (400) is carried out from movement data generated by the inertial units (21) while they are positioned in an electronic casing arranged to be fixed on the shoe worn by said individual.

**5.** The method (1) for inferring a plantar pressure line (30) according to any one of claims 1 to 3, **characterized in that** the calculation step (400) is carried out from movement data generated by the inertial units (21) while they are positioned in an electronic casing, said electronic casing also including the one or several microprocessors (22) configured to calculate the foot angle values relative to the predetermined reference frame.

**6.** The method (1) for inferring a plantar pressure line (30) according to the preceding claim, **characterized in that** the calculation (400) of foot angle values and the inference (500) of a plurality of pressure centers are implemented by one or several microprocessors (22) positioned in an electronic casing integrated into a sole.

**7.** The method (1) for inferring a plantar pressure line (30) according to any one of claims 1 or 2, **characterized in that** the calculation step (400) is performed from movement data generated by the inertial units (21) while they are positioned at three different locations on the foot of said individual.

8. The method (1) for inferring a plantar pressure line (30) according to any one of the preceding claims, **characterized in that** the calculation (400) of foot angle values includes the calculation of values of at least two angles of the foot.

9. The method (1) for inferring a plantar pressure line (30) according to any one of the preceding claims, **characterized in that** said predetermined reference frame is external to the human body and is selected from: the ground, a calculated gait line, or a calculated trajectory of said foot of said individual.

10. The method (1) for inferring a plantar pressure line (30) according to the preceding claim, **characterized in that** the calculated foot angle values, relative to the predetermined reference frame, include angle values selected from: an angle of the antero-posterior axis of the foot relative to its progression line, an angle of the antero-posterior axis of the foot relative to the ground, an angle of the transverse axis of the foot relative to its progression line or an angle of the transverse axis of the foot relative to the ground or the strike angle between the base of the foot and the ground at initial contact..

11. The method (1) for inferring a plantar pressure line (30) according to the preceding claim, **characterized in that** it includes a comparison of the plantar pressure lines (30) of each of the feet of the individual.

12. The method (1) for inferring a plantar pressure line (30) according to any one of the preceding claims, **characterized in that** it includes a comparison of the plantar pressure lines (30) obtained for the same foot for two different time intervals.

13. The method (1) for inferring a plantar pressure line (30) according to any one of the preceding claims, **characterized in that** it includes a step of preprocessing the movement data generated by the at least one inertial unit (21), the preprocessing step including frequency filtering, gravity suppression, and/or drift suppression.

14. A device for inferring (2) a plantar pressure line (30), said plantar pressure line (30) corresponding to the time evolution of the position of the barycenter of plantar pressures during the movement of an individual and including a plurality of successive positions of said barycenter of plantar pressures for a foot of said individual, said inference device (2) including one or several microprocessors (22), said one or several microprocessors (22) being configured to:

- load a correlation model, said correlation model defining a relationship between a plurality of data obtained from plantar pressure sensors and a plurality of foot angle values relative to a predetermined reference frame, said relationship enabling the determination of the position of the barycenter of plantar pressures at each instant of a stance phase;
- calculate foot angle values relative to the predetermined reference frame, from movement data generated by at least one inertial unit (21), over at least two instants of the stance phase of the foot of said individual; and
- infer a plurality of successive positions of the barycenter of the plantar pressures to form the plantar pressure line (30) of said individual's foot, from the angle values calculated over the at least two instants of the stance phase of the foot and from the model correlation.

15. The device for inferring (2) a plantar pressure line (30) according to the preceding claim, **characterized in that** it further includes, the at least one inertial unit (21) coupled to a shoe worn by said individual or to said individual's foot, said at least one inertial unit (21) being configured to generate movement data for a plurality of instants of a stance phase of a foot of said individual.

1

200 — Chargement d'un modèle de corrélation

400 — Calcul de valeurs d'angles du pied sur au moins deux instants de la phase d'appui d'un pied d'un utilisateur

500 — Inférence de la ligne de pression plantaire du pied de l'individu à partir des valeurs d'angles calculées et du modèle de corrélation

## FIG. 1

100

100 — Apprentissage

200 — Chargement d'un modèle de corrélation basé sur des données de capteur de pression acquises lors de la marche

300 — Génération de données de mouvement à partir d'au moins une centrale inertielle

350 — Prétraitement des données de mouvement

400 — Calcul de valeurs d'angles du pied sur au moins deux instants de la pose du pied de l'utilisateur

500 — Inférence de la ligne de pression plantaire inférée de l'individu à partir des valeurs d'angles calculées

600 — Comparaison de lignes de pression plantaire inférées

700 — Transmission de lignes de pression plantaire inférées

800 — Mémorisation de lignes de pression plantaire inférées

**FIG. 2**

FIG. 3

FIG. 4

**FIG. 5**

**FIG. 6**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2019077266 A
- WO 2019193301 A
- WO 2020217037 A
- US 20090163834 A
- CN 108209924
- CN 111631719
- WO 2012026818 A
- WO 2013022344 A

**Littérature non-brevet citée dans la description**

- **MICHAELA ANNE A. DELA CRUZ et al.** Joint Gait Kinematic and Kinetic Analysis using Inertial Measurement Units and Plantar Pressure Sensor System. *Conference 2019 IEEE.*
- **MAHDAVIAN MOHAMMAD et al.** Motion Generation of a Wearable Hip Exoskeleton Robot Using Machine Learning-Based Estimation of Ground Reaction Forces and Moments. *2019 IEEE/ASME INTERNATIONAL CONFERENCE ON ADVANCED INTELLIGENT MECHATRONICS (AIM)*
- **JANIN M.** Marche athlétique : modification des pressions plantaires par éléments d'orthèse. *Cinésiologie*, 2002, vol. 203, 13-4